Europäisches Patentamt

European Patent Office

Office européen des brevets

(19)

(11) Veröffentlichungsnummer: **0 151 246 B1**

(12) **EUROPÄISCHE PATENTSCHRIFT**

(45) Veröffentlichungstag der Patentschrift: **19.09.90**

(21) Anmeldenummer: **84114064.3**

(22) Anmeldetag: **22.11.84**

(51) Int. Cl.⁵: **C 07 K 7/00,** C 07 K 7/10, C 12 P 21/02 // C12P7/64, C12P13/00 ,(C12P21/02, C12R1:465)

(54) **Neue Polypeptide mit alpha-Amylase-hemmender Wirkung, Verfahren zu deren Herstellung, deren Verwendung und pharmazeutische Präparate.**

(30) Priorität: **21.01.84 DE 3402021**

(43) Veröffentlichungstag der Anmeldung: **14.08.85 Patentblatt 85/33**

(45) Bekanntmachung des Hinweises auf die Patenterteilung: **19.09.90 Patentblatt 90/38**

(84) Benannte Vertragsstaaten: **AT BE CH DE FR GB IT LI LU NL SE**

(56) Entgegenhaltungen:
**EP-A-0 049 847**
**DE-A-2 716 050**

**CHEMICAL ABSTRACTS, Band 90, 1979, Seite 221, Zusammenfassung Nr. 2229p, Columbus, Ohio, US; E. HOSTINOVA et al.: "Alpha-Amylase from Streptomyces aureofaciens - purification and properties", & STAERKE 1978, 30(10), 338-41**

(73) Patentinhaber: **HOECHST AKTIENGESELLSCHAFT Postfach 80 03 20 D-6230 Frankfurt am Main 80 (DE)**

(72) Erfinder: **Vértesy, László, Dr. Eppenhainer Weg 6 D-6239 Eppstein (Taunus) (DE)**
Erfinder: **Tripier, Dominique, Dr. Im Kirschgarten 16 D-6239 Eppstein (Taunus) (DE)**
Erfinder: **Ritzel, Harald, Dr. An der Hayl 4 D-6500 Mainz 43 (DE)**

(56) Entgegenhaltungen:
**CHEMICAL ABSTRACTS, Band 92, 1980, Seite 228, Zusammenfassung Nr. 159613y, Columbus, Ohio, US; E. HOSTINOVA et al.: "The action of extracellular alpha-amylase from Streptomyces aureofaciens on starch", BIOLOGICA (BRATISLAVA) 1980, 35(3), 191-4**
**Biol. Chem. Hoppe-Seyler, Vol. 366, S. 1161-1168**

**Beschreibung**

Die Erfindung betrifft neue, biologisch aktive cyclische Polypeptide. Sie besitzen α-Amylase-hemmende Eigenschaften und können daher in der Human- und Tiermedizin, in der medizinischen Diagnostik sowie in der Biotechnologie der Stärke verwendet werden.

α-Amylase-Inhibitoren sind bereits bekannt. So wird in der deutschen Offenlegungsschrift 2716050 ein α-Amylase-Inhibitor und seine Gewinnung mittels eines Streptomyceten (ATCC 31209) beschrieben. In der europäischen Patentanmeldung EP 0049847 wird ein α-Amylaseinaktivator, der aus zwei Komponenten besteht sowie seine Herstellung mittels Streptomyceten (ATCC 31210, DSM 1912) offenbart.

Die α-Amylase-hemmenden Polypeptide der vorliegenden Erfindung sind nun dadurch gekennzeichnet, daß sie aus 17 bis 200 Aminosäuren bestehen und folgende Formel I aufweisen

$$\overbrace{\underset{Z - S - Trp - Arg - Y}{\underbrace{\phantom{xxxxxxxxxxxx}}}}^{AS} \qquad\qquad I$$

in welcher bedeuten:

Z: Gln,

S: Ser,

Y: Tyr,

AS: eine Polypeptidkette aus 12 natürlichen Aminosäuren, die ihrerseits durch 2 Peptidketten substituiert ist, wobei der α-Amylaseinaktivator Tendamistat (HOE 467) sowie der aus dem Kulturfiltrat von Streptomyces violaceoruber ATCC 31209 gewonnene α-Amylaseinhibitor ausgenommen sind.

Von den erfindungsgemäßen Polypeptiden sind das Peptid der Formel Ia

```
    Pro-Ala-Pro-Asp        Ser-Asp-Ala-Val-Thr-Val-Val
    |   5                   |         /                 30      |
    Ser           Val-Cys-Cys-Gly-Asn-Arg                     Val
    |             |  10     25               |                  |
    Gly           Glu                       Val                Gln        Ia
    |             |                          |                  |
    |             Ser                      20Asp                |
    Thr           Phe   15                  Thr               35Tyr
    |             |                          |                  |
    |             Gln— Ser— Trp— Arg —Tyr                       |
    Ala1                                                       Glu
```

das in den folgenden Ausführungen auch als AI—3688 bezeichnet wird, sowie die folgenden Strukturvarianten bevorzugt:

Das 1-Desalanin-Derivat AI—3688a (die Aminosäure 1 ist nicht Ala sondern Thr),

das Peptid AI—3688b, das sich von IA—3688 dadurch unterscheidet, daß die 14. Aminosäure Gln ausgetauscht wurde durch Glu,

das Peptid AI—3688c, das sich von AI—3688 dadurch unterscheidet, daß die 34. Aminosäure Gln ausgetauscht wurde durch Glu,

das Peptid AI—3688d, das sich von AI—3688a dadurch unterscheidet, daß die 13. Aminosäure Gln ausgetauscht wurde durch Glu (dieses Peptid ist das 1-Desalanin-Derivat von AI—3688b),

das Peptid AI—3688e, das sich von AI—3688a dadurch unterscheidet, daß die 33. Aminosäure Gln ausgetauscht wurde durch Glu (dieses Peptid ist das 1-Desalanin-Derivat von AI—3688c),

das Peptid AI—3688f, das sich von AI—3688 dadurch unterscheidet, daß sowohl die 14. als auch die 34. Aminosäure Gln ausgetauscht wurden durch Glu, und das Peptid AI—3688g, das das 1-Desalanin-Derivat von AI—3688f ist.

Bei diesen Polypeptiden bedeutet Z Gln, S Ser, Y Tyr und AS eine Polypeptidkette aus 12 Aminosäuren, die mit 2 Peptidketten aus 8 oder 7 bzw. 11 Aminosäuren disubstituiert ist. Die Peptide weisen 36 bzw. 35 Aminosäuren auf. Das Aminoterminale Ende der Aminosäurensequenz befindet sich am Alanin (1) bzw. Thr. Bei dem Peptid AI—3688 besteht zwischen dem Cys (9) und Cys (25) eine Disulfidbrücke. Die Disulfidbrücken befinden sich bei den Varianten an entsprechenden Stellen.

In den vorstehenden und folgenden Ausführungen werder die im Inhibitor enthaltenen Aminosäuren durch Symbole beschrieben, wie sie im Buch von L. Styer "Biochemistry", W. H. Freeman & Company, San Francisco, 1972, Seite 14ff, verwendet werden.

**EP 0 151 246 B1**

Die Erfindung betrifft ferner ein Verfahren zur Herstellung der cyclischen Polypeptide der Formel I, pharmazeutische Präparate enthaltend eine Verbindung der Formel I, die Verwendung als Arzneimittel, Diagnostikum und Reagenz.

Insbesondere betrifft die Erfindung Verfahren zur Herstellung des Polypeptids AI—3688 sowie der Varianten AI—3688 a bis g, pharmazeutische Präparate enthaltend AI—3688 oder eines der Varianten und ihre Verwendung als Arzneimittel, Diagnostikum und Reagenz.

Das Verfahren zur Herstellung der Polypeptide der Formel I ist dadurch gekennzeichnet, daß man einen Polypeptide der Formel I erzeugenden Streptomycetes in einem Fermentationsmedium im Submersverfahren kultiviert, das Polypeptid aus dem Mycel oder dem Kulturfiltrat in an sich bekannter Weise isoliert und reinigt. Von den Streptomyceten ist Streptomyces aureofaciens insbesondere geeignet.

AI—3688 und die Strukturvarianten AI—3688 a-g werden vorzugsweise aus dem Mycel und Kulturfiltrat von Streptomyces aureofaciens FH 1656 gewonnen. Dieser Stamm ist bei der Deutschen Sammlung von Mikroorganismen (DSM) unter der Registrier-Nr. DSM 2790 hinterlegt worden. Es können aber auch die Varianten und Mutanten dieses Stammes zur Gewinnung von AI—3688 und der Strukturvarianten eingesetzt werden.

Die taxonomischen Eigenschaften des Streptomyces aureofaciens DSM 2790 entsprechen der Beschreibung von Streptomyces aureofaciens nach dem Bergley's Manual of Determinative Bacteriology, 8. Auflage, Verlag Williams & Wilkins Corp. Baltimore, 1974. Der Unterschied zu den beschriebenen Stämmen besteht in dem Stoffwechselprodukt. Cyclische Peptide als Stoffwechselprodukte von Streptomyces aureofaciens-Stämme wurden bisher nicht beschrieben. Folglich ist der Stamm neu. Die Erfindung betrifft daher auch Streptomyces aureofaciens DSM 2790.

Bei der Gewinnung von AI—3688 geht man zweckmäßig wie folgt vor:

Streptomyces aureofaciens DSM 2790 wird in einem wäßrigen Nährmedium unter submersen und vorzugsweise aeroben Bedingungen gezüchtet, bis man eine ausreichende Konzentration des AI—3688 erhält. Das Nährmedium enthält einerseits Kohlenstoffquellen, wie beispielsweise Kohlenhydrate, andererseits Stickstoffquellen, wozu geeignete Stickstoffverbindungen zählen, wie z.B. proteinhaltige Materialien. Bevorzugte Kohlenstoff liefernde Verbindungen sind Glucose, Saccharose, Glycerin, Malzextrakt, Stärke, Öle, Fette und dergleichen. Bevorzugte Stickstoff liefernde Substanzen sind beispielsweise Maisquellwasser, Hefeextrakte, Sojamehl, Fischmehl, Magermilchpulver, angedautes Casein oder Fleischextrakt. Es können auch sogenannte "synthetische" Nährlösungen verwendet werden. Es kann weiterhin nützlich sein, Spurenelemente, wie z.B. Zink, Magnesium, Eisen, Kobalt oder Mangan dem Fermentationsmedium zuzugeben.

Die Fermentation, die zur Bildung des AI—3688 führt, kann in einem weiten Temperaturebereich durchgeführt werden. Beispielsweise wird sie bei Temperaturen zwischen 10 und 40°C, vorzugsweise zwischen ungefähr 25 und 35°C durchgeführt. Den pH-Wert des Mediums hält man ebenfalls bei Werten, die dem Wachstum der Mikroorganismen förderlich sind, beispielsweise bei Werten zwischen 4,0 und 9,0, vorzugsweise zwischen 5,0 und 8,0. In Abhängigkeit vom Nährmedium, wie z.B. seiner qualitativen und quantitativen Zusammensetzung und den Fermentationsbedingungen, wie z.B. Belüftungsrate, Temperatur oder pH-Wert, erfolgt die Bildung des AI—3688 in der Kulturlösung gewöhnlich nach etwa 2—10 Tagen.

Der AI—3688 befindet sich sowohl im Mycel als auch im Kulturfiltrat der Fermentation. Die Hauptmenge des AI—3688 ist im allgemeinen im Kulturfiltrat zu finden. Deshalb wird vorteilhafterweise die wäßrige Phase vom Mycel, beispielsweise durch Filtration oder Zentrifugation getrennt und das AI—3688 aus den jeweiligen Phasen durch an sich bekannte Verfahren isoliert und gereinigt. Hierzu eignet sich eine große Zahl von Verfahren, wie beispielsweise Chromatographie an Ionenaustauschern, Molekularsieben oder Adsorptionsharzen, Kristallisation, Lösungsmittel- bzw. Salzfällungen, Ultrafiltration, Craig-Verteilung u.a.

Ein bevorzugtes Verfahren zur Gewinnung des AI—3688 besteht darin, daß man den Inhibitor aus dem Kulturfiltrat an ein geeignetes Harz wie z.B. auf Polystyrolbasis adsorbiert, dieses beladene Harz abtrennt und den Inhibitor AI—3688 durch Elution mit geeigneten Pufferlösungen wie z.B. Phosphat- oder Bicarbonatpufferlösung oder mit ggfs. wasserhaltigen organischen Lösungsmitteln, wie z.B. Methanol, Ethanol, Aceton vorzugsweise aber mit wäßrigem Isopropanol isoliert. Die inhibitorhaltigen Eluate konzentriert man durch Ultrafiltration in bekannter Weise, wobei gleichzeitig eine Entsalzung vorgenommen wird. Die ionenarme wäßrige Lösung des AI—3688 wird dann auf einer Ionenaustauscher Säule chromatographisch in an sich bekannter Weise aufgetrennt werden. Vorzugsweise verwendet man DEAE-Cellulose oder DEAE-modifizierte Cellulose (z.B. DEAE-®Sephadex) als Ionenaustauscher, brauchbar sind aber auch eine große Anzahl anderer käuflicher Kationen- und Anionenaustauscher. Der letzte Schritt der Isolierung ist die Anwendung eines Molekularsiebes (z.B. ®Biogel P-6 oder ®Sephadex). Die resultierenden wäßrigen Lösungen des reinen Materials werden dann z.B. durch Lyophilisierung getrocknet. Die spezifische Aktivität beträgt $1,5 \times 10^4$ α-Amylaseinhibitoreinheiten per mg Festsubstanz.

Der reine Inhibitor AI—3688 ist ein farbloses Polypeptid, dessen Aminosäurenanalyse die Anwesenheit der meisten natürlichen Aminosäuren zeigt. Er hat die oben angegebene Formel Ia.

Es kann bei der Fermentation, insbesondere bei längerer, vorkommen, daß der Inhibitor AI—3688 nicht einheitlich ist. In diesen Fällen kann eine Auftrennung in die einzelnen Komponenten mit Hilfe der HPLC-Technik durchgeführt werden, vorzugsweise mittels HPLC auf Reversed Phase RP 18 Trägern mit einem

3

Wasser-Acetonitril Gemisch (95:5) als Elutionsmittel. Die sogewonnenen Komponenten haben die angegebenen Strukturen AI—3688 a, b, c, d, e, f bzw. g. Die Strukturvarianten sind gleichermaßen wirksam wie AI—3688.

Es ist für AI—3688 und AI—3688 a bis g kennzeichnend, daß die natürlichen Aminosäuren Met, Ile, Leu, Lys und His fehlen.

Das ultraviolette Licht wird in Wasser vom AI—3688 absorbiert mit $\lambda_{max} = 275$ nm, $E_{1cm}^{1\%} = 21$. Der isoelektrische Punkt beträgt — bestimmt durch isoelektrische Fokusierung — 4,2. Der Inhibitor AI—3688 ist in Wasser und wäßrigen Pufferlösungen leicht löslich, lediglich in der Nähe des isoelektrischen Punktes nimmt die Löslichkeit ab.

Durch die o.g. Aminosäurenzusammensetzung und natürlich durch die Struktur unterscheiden sich AI—3688 sowie AI—3688 a-g von allen bekannten α-Amylaseinhibitoren, somit liegen hier neue Substanzen vor.

Die chemische Struktur — wie in Formel I a wiedergegeben — zeigt die Aminosäurensequenz des AI—3688 sowie darüber hinaus das vorliegen eines Ringes, der von 17 Aminosäuren durch die Verknüpfung des Cys (Position 9) mit dem Cys (Position 25) gebildet wird. Nach P. Y. Chou und G. D. Fasmann (Advances in Enzymology, Band 47, Seite 45—148, erschienen bei John Wiley & Son, 1978) erlaubt die Primärstruktur — aufgrund der räumlichen Eigenarten der einzelnen beteiligten Aminosäuren und ihrer Verknüpfung, d.h. ihrer Sequenz und ihrer Ringbildung — Rückschlüsse auf die Sekundärstruktur der Peptide. Es wurde mit dem Inhibitor AI—3688 eine Chou/Fasman-Analyse durchgeführt. Dabei ist überraschend gefunden worden, daß die Teilsequenz des Rings: *-Gln-Ser-Trp-Arg-Tyr-* an exponierter Stelle, d.h. räumlich hervorragend, für biochemische Reaktionen in geeigneter Weise in das Molekül eingebaut und letztlich für die Wirksamkeit verantwortlich ist. Es ist weiter gefunden worden, daß durch Öffnen des 17 Aminosäuren enthaltenden Ringes, z.B. durch Lösen der Schwefel-Schwefel-Bindung des Cystins eine Veränderung der räumlichen Struktur bewirkt wird, wodurch die obengenannte entscheidende Teilsequenz dann nicht mehr räumlich hervortritt und die enzymhemmende Wirksamkeit verloren geht. Durch Ringöffnung entstandene chemische Derivate des α-Amylaseinhibitors AI—3688 sind biologisch und biochemisch inaktiv (vgl. Beispiel 4). Charakteristisch für die erfindungsgemäßen α-Amylaseinaktivatoren und wesentlich für ihre Hemmwirkung ist daß ein großer, aus 10—30 vorzugsweise aus 17 Aminosäuren bestehender Ring vorliegt und in diesem Ring die Aminosäurensequenz -Z-S-Trp-Arg-Y-, insbesondere *-Gln-Ser-Typ-Arg-Tyr-* enthalten ist. Die Zusammensetzung der Teilsequenz wie sie die erfindungsgemäßen Polypeptide aufweisen beeinflußt die Stärke der enzymhemmenden Wirksamkeit wenig. Auch ist die Natur der Ringbildung des cyclischen Peptids nicht wesentlich: weder die chemische Bindungsart der Cyclisierung noch der Weg, d.h. ob biologisch oder synthetisch gewonnen, ist für die Wirksamkeit bedeutend, wichtig ist das Vorliegen eines Ringsystems, welches die angegebenen Aminosäurenteilsequenzen enthält.

Untersuchungen mit dem in der Deutschen Offenlegungsschrift 27 16 050 beschriebenen, aus Streptomyces violaceoruber ATCC 31209 isolierten α-Amylaseinaktivator zeigten, daß die angegebene Teilsequenz sowie das Vorliegen eines Ringsystems für die Hemmwirkung verantwortlich sind. Die Untersuchung wurde wie folgt durchgeführt.

Bestimmung der Teilsequenz des α-Amylaseinhibitors gewonnen aus Streptomyces violaceoruber ATCC 31209: Die Sequenzierung des durch reduktive oder oxidative Ringöffnung linearisierten α-Amylaseaktivators erfolgte mit dem Beckmann-Sequenator® in 0,2 molarem Puffersystem (Quadrol®) automatisch mit der Aminosäurenphenylthiohydantoin (PTH) Methode. Zwischen den beiden Cysteinresten der Substanz wurde u.a. folgende Teilsequenz gefunden: -Tyr-Phe-*Gln-Ser-Trp-Arg-Tyr*-Thr-Asp-Val-His-.

Die Eigenschaften der erfindungsgemäßen Inhibitoren sind im Hinblick auf die Anwendung als Therapeutikum gegen Diabetes und Prädiabetes sowie Adipositas und Unterstützung von Diät interessant. Aufgrund ihrer Eigenschaften sind sie auch als Reagenz für diagnostische Zwecke wertvoll.

Stärkehaltige Nahrungs- und Genußmittel führen bei Tier und Mensch zu einem Anstieg des Blutzuckers und dadurch auch zu einer vermehrten Insulinsekretion des Pankreas. Die Hyperglykämie kommt durch die Aufspaltung der Stärke im Verdauungstrakt unter Einwirkung von Amylase und Maltase zu Glukose zustande.

Bei Diabetikern ist die Hyperglykämie besonders ausgeprägt und langanhaltend.

Die alimentäre Hyperglykämie sowie die Hyperinsulinämie nach Stärkeaufnahme läßt sich durch die erfindungsgemäßen Amylase-Inhibitoren insbesondere durch AI—3688, vermindern. Diese Wirkung ist dosisabhängig. Die erfindungsgemäßen Amylase-Inhibitoren lassen sich daher als Therapeuticum einsetzen bei Diabetes, Prädiabetes und Adipositas sowie zur Unterstützung von Diät. Zu diesem Zweck empfiehlt sich eine Verabreichung, insbesondere zu den Mahlzeiten. Die Dosierung, die sich nach dem Gewicht des Patienten sowie dem individuellen Bedarf ausrichten soll, beträgt etwa 10—500 mg pro Dosis, die zweckmäßig zu jeder Mahlzeit genommen wird. Die Dosierung kann jedoch in begründeten Einzelfällen auch darüber oder darunter liegen.

Die erfindungsgemäßen Amylase-Inhibitoren eignen sich insbesondere zur oralen Verabreichung. Sie können als reine Substanz aber auch in Form einer pharmazeutischen Zubereitung unter Verwendung der üblichen Hilfs- und Trägerstoffe angewandt werden. Auch eine kombinierte Anwendung mit anderen Arzneimitteln wie blutzuckersenkenden oder lipidsenkenden Substanzen kann von Vorteil sein. Da höhermolekulare Peptide als soche nicht oder nicht nennenswert aus dem Verdauungstrakt resorbiert werden, sind von den erfindungsgemäßen Substanzen keine toxikologisch bedenklichen Nebenwirkungen zu erwarten. Aufgrund der nicht außergewöhnlichen Aminosäurezusammensetzung sind auch evtl. proteolytische Spaltprodukte als physiologisch unbedenklich anzusehen. Dementsprechend konnten bei oraler Gabe auch hoher Dosen des Amylaseinhibitors AI—3688 an Versuchstiere keine auffälligen Symptome erkannt werden. Zur Prüfung der pharmakologischen Wirkung des Amylase-Inhibitors erhielten nüchterne, männliche Wistar-Ratten mit einem Gewicht zwischen 200 und 250 g den erfindungsgemäßen Hemmstoff AI—3688 gleichzeitig mit 2 g Stärke pro kg Körpergewicht oral verabreicht. Durch Bestimmung von Blutzuckerkonzentrationen in Blutproben, die vor, während und nach der Applikation des α-Amylaseinhibitors entnommen worden sind, wurde die Wirksamkeit des Präparates bewiesen.

Neben der Blutglukoseregulation kann man die erfindungsgemäßen Polypeptide auch zur Hemmung der Speichel-α-Amylase verwenden. Dieses Enzym bewirkt die Verdauung der Stärke im Mund und der so gebildete Zucker fördert den Kariesbefall der Zähne. Die erfindungsgemäßen Verbindungen können daher zur Verhütung oder Verminderung der Ausbildung von Karies angewandt werden.

Weiterhin können sie als biochemische Reagenzien sowie als diagnostische Mittel verwendet werden.

Amylasetest

Ein Amylase-Inhibitoreinheit (AIE) ist definiert als die Inhibitormenge, die unter den Testbedingungen zwei Amylase-Einheiten (AE) zu 50% zu hemmen vermag. Eine Amylase-Einheit ist nach internationaler Übereinkunft die Enzymmenge, die in einer Minute 1 μ äquivalent glucosidischer Bindungen in der Stärke spaltet. Die μVal gespaltener glucosidischer Bindungen werden als μVal reduzierender Zucker photometrisch mit Dinitrosalizylsäure bestimmt. Die Angaben sind als μMole Maltose berechnet, die anhand einer Maltose-Eichgeraden ermittelt werden.

Die Tests werden folgendermaßen durchgeführt:

α-Amylase aus Schweinepankreas und die zu testenden Lösungen werden gemeinsam in 1,0 ml 20 mM Phosphatpuffer, pH $6,9_1$ + 10 mM NaCl 10—20 min bei 37°C vorinkubiert. Die enzymatische Reaktion wird durch Zugabe von 1,0 ml löslicher Stärke (0,25% in dem angegebenen Puffer) Nach Zulkowski gestartet. Nach genau 10 min wird die Reaktion mit 2,0 ml Dinitrosalizylsäure-Farbreagenz (nach Boehringer Mannheim: Biochemica-Information II) abgestoppt und zur Farbentwicklung 5 min im siedenden Wasserbad erhitzt. Nach dem Abkühlen wird die Extinktion bei 546 nm gegen den Reagentienleerwert gemessen. Die 50%ige Hemmung wird im Vergleich zur ungehemmten Enzymreaktion durch Einsatz verschiedener Inhibitormengen graphisch mittels der Wahrscheinlichkeitsauftragung bestimmt.

Beispiel 1

Zur Gewinnung von AI—3688 erfolgte die Impfstoffanzucht (Anzucht des Mikroorganismus) — wie in der mikrobiologischen Praxis üblich — aus einer gefriergetrockneten Dauerform des Organismus Streptomyces aureofaciens FH 1656, DSM 2790, über Einzelkoloniepassage und Schrägröhrchen. Die für die Fermentation notwendige Massenproduktion an Sporen wurde ebenfalls auf festem Nährboden in Roux-Flaschen durchgeführt.

*Agarmedium für Platte, Schrägröhrchen und Roux-Flasche:*

| | |
|---|---|
| Dextrin | 15,0 g/l |
| Rohrzucker | 3,0 g/l |
| Fleischextrakt | 1,0 g/l |
| Hefeextrakt | 2,0 g/l |
| Kochsalz | 0,5 g/l |
| $K_2HPO_4$ | 0,5 g/l |
| $FeSO_4 \times 7 H_2O$ | 0,01 g/l |
| Agar-Agar | 2,0 g/l |
| pH-wert | 7,3 |

Sterilisation bei 120°C 20 min.
Inkubation bei 30°C 9 Tage.

Mit der Sporenabschwemmung von der Roux-Flasche in 100 ml sterilem Wasser wurde eine vegetative Fermentationsvorstufe in einer Fernbachflasche beimpft (Arbeitsvolumen 1,2 l).

5

*Vorstufenmedium:*

| | |
|---|---|
| Glukose | 30,0 g/l |
| Sojamehl | 20,0 g/l |
| Maisstärke | 2,0 g/l |
| Harnstoff | 1,0 g/l |
| Ammoniumnitrat | 1,0 g/l |
| Malzextrakt | 5,0 g/l |
| pH-Wert | 6,8 |

Sterilisation 120°C 20 min.
Inkubation bei 28°C 2 Tage auf einer Schüttelmaschine mit 150 UpM und 5 cm Amplitude.

*Hauptfermentation:* Nach 48 Stunden Vorkultur (s.o.) wurden 200 l Hauptfementationsmedium mit 1,2 l Impfstoff aus der Fernbach-Flasche inokuliert.

*Medium:*

| | |
|---|---|
| lösliche Stärke | 4,0 g/l |
| Glukose | 1,0 g/l |
| Casein-Pepton | 1,0 g/l |
| Cornsteep (flüssig) | 0,4 g/l |
| Sojamehl | 0,4 g/l |
| $(NH_4)_2HPO_4$ | 0,8 g/l |
| pH-Wert | 8,3 |

Sterilisation 120°C 30 min.

Die Inkubation erfolgte bei 28°C unter Rühren mit einer Umfanggeschwindigkeit von 5 m/sec und 0,1 vvm Belüftungsrate während 5 Tagen. Der pH-Wert fiel während der Fermentation ab und wurde mit Natriumhydroxid auf 5,5—6,0 gesteuert. Nach 5 Tagen wurde abgeerntet, die Ausbeute betrug 60 mg AI—3688 pro Liter Kulturlösung.

### Beispiel 2

180 l Fermentationslösung gemäß Beispiel 1 wurden mit Hilfe einer Zentrifuge von der Zellmasse befreit und die klare flüssige Phase auf pH 4,9 eingestellt. Anschließend trug man die Lösung auf eine 15 l Polystyrol-Adsorptionsharz (Diaion® HP-20) beeinhaltende Säule auf, wusch mit 30 l Wasser nach und eluierte mit Wasser, dem steigende Mengen Isopropanol hinzugefügt worden war. Die Mischung, die 25% Isopropanol enthielt, löste den Inhibitor AI—3688 von der Säule. Diese aktiven Eluate (25 l) wurden durch Ultrafiltration konzentriert und unter Wasserzugabe sowie weitere Ultrafiltration solange entsalzt, bis das Retentat keine nachweisbaren Salze mehr enthielt. Das resultierende Konzentrat (1,5 l) trennte man auf DEAE-modif. Cellulose (DEAE-Sephadex®), welche mit 1/15 U Phosphatpuffer, pH 5,6, equilibriert worden war. Durch Anlegen eines zusätzlichen Kochsalzgradienten wurde die α-Amylaseaktivität eluiert. Die entsprechenden Fraktionen wurden entsalzt. Man konzentrierte in Ultrafiltrationszellen (Amicon®), welche 1/15 U Phosphatpuffer, pH 5,6, equilibriert worden war. Durch Anlegen eines zusätzlichen Kochsalzgradienten wurde die α-Amylasehemmaktivität eluiert. Die entsprechenden Fraktionen wurden entsalzt. Man konzentrierte in Ultrafiltrationszellen (Amicon®) erneut. Die Endreinigung geschah an Polyacrylamid-Gel (Biogel P-6®) mit reinem Wasser als Laufmittel. Die AI—3688-haltigen Fraktionen dieser Säule wurden gesammelt und gefriergetrocknet. Es resultierten 1,2 g eines farblosen Pulvers mit $1,5 \times 10^4$ α-Amylaseinhibitoreinheiten pro mg.

### Beispiel 3

Die Gewinnung eines mehrere Komponenten enthaltenden AI—3688-Inhibitorkomplexes geschah wie in Beispiel 1 beschrieben, jedoch wurde die dort angegebene Fermentationszeit von 5 Tagen auf 8 Tage ausgedehnt. Die Aufarbeitung der Fermentationslösung erfolgte nach Beispiel 2, wobei aber die resultierende farblose Substanz (0,9 g) chemisch nicht einheitlich war. 100 mg dieses Pulvers wurden in 1 ml Wasser/Acetonitril (95:5 v/v) gelöst und auf eine Stahlsäule (mit den Innenmaßen 3,2 × 25 cm$^2$) aufgetragen, die mit reversed phase-Kieselgel RP$_{18}$ gefüllt war. Die Elution geschah mit dem Lösungsmittelgemisch Wasser-Acetonitril (95:5), welches mit Trifluoressigsäure auf pH 2,1 eingestellt war. Bei einem durch Kolbenpumpen erzeugten Arbeitsdruck von 70 Bar stellte sich eine Durchflußgeschwindigkeit von 8 ml pro Minute ein. Den Säulenausfluß kontrollierte man durch Bestimmung der Ultraviolett-absorption bei 276 nm. Nach 41 bis 43 Minuten wurden mehrere absorbierende Verbindungen von der Säule gewaschen, die alle getrennt gesammelt, auf α-Amylasehemmende Wirkung getestet, im Vakuum eingeengt und anschießend gefriergetrocknet wurden. Die einzelnen Fraktionen wurden mit einem Beckman Sequencer 890 C auf Einheitlichkeit untersucht und die Aminosäuresequenz bestimmt.

Hierbei ergab die Analyse von

Peak 1 eine Aminosäuresequenz, deren Struktur der Formel 1a entsprach, wobei jedoch die Aminosäure 34 (Glu) gegen Gln ausgetauscht war (=AI—3688c)

Peak 2 eine Aminosäuresequenz, deren Struktur der Formel Ia entsprach (=AI—3688)

Peak 3 eine Aminosäuresequenz wie in der Formel I a beschrieben, jedoch war die Aminosäure 14 (Gln) gegen Glu ersetzt (=AI—3688b)

Peak 4 1-Des-Ala-AI—3688 (=AI—3688a)

Peak 5 eine Mischung von zwei 1-Des-Ala-AI—3688-Verbindungen; in einer war die Aminosäure 33 (Glu) durch Gln ersetzt (=AI—3688e) in der anderen die Aminosäure 13 (Gln) durch Glu (=AI—3688d).

Die spezifischen α-Amylase-Hemmaktivitäten aller Verbindungen betrugen $(1,5 \pm 0,1) \times 10^4$ AIE per mg.

Beispiel 4

(Ringspaltung, Nachweis des wirksamen Prinzips)

10 mg AI—3688 gem. Beispiel 2 wurden in 5 ml Phosphatpuffer, pH 7,0, gelöst. Eine kleine Probe wurde zum Testen entnommen und anschließend mit 10 mg Dithioerythrit versetzt. Nach 18 Stunden Standzeit bei Raumtemperatur wurde die Reaktionslösung im Vergleich zur Ausgangslösung auf α-Amylasehemmende Wirksamkeit getestet. Während die Ausgangssubstanz mit $1,5 \times 10^4$ AEl pro mg voll aktiv war, war die Wirksamkeit der Reaktionslösung unterhalb der Nachweisgrenze.

**Patentansprüche für die Vertragsstaaten: BE CH DE FR GB IT LI LU NL SE**

1. Cyclische Polypeptide, dadurch gekennzeichnet, daß sie aus 17 bis 200 Aminosäuren bestehen und folgende Formel I aufweisen

$$Z - S - Trp - Arg - Y \qquad\qquad I$$

in welcher bedeuten:

Z: Gln,

S: Ser,

Y: Tyr,

AS: eine Polypeptidkette aus 12 natürlichen Aminosäuren, die ihrerseits mit 2 Peptidketten substituiert ist,

wobei der α-Amylaseinaktivator Tendamistat (HOE 467) sowie der aus dem Kulturfiltrat von Streptomyces violaceoruber ATCC 31209 gewonnene α-Amylaseinhibitor ausgenommen sind.

2. Cyclische Polypeptide gemäß Anspruch 1, dadurch gekennzeichnet, daß es die Formel Ia

```
Pro-Ala-Pro-Asp        Ser-Asp-Ala-Val-Thr-Val-Val
     |    5           |          /            30        |
    Ser          Val-Cys-Cys-Gly-Asn-Arg              Val
     |             |  10      25        |               |
    Gly           Glu                  Val             Gln        Ia
     |             |                    |               |
     |            Ser                20Asp              |
    Thr           Phe   15            Thr            35Tyr
     |             |                    |               |
    Ala1         Gln— Ser— Trp- Arg —Tyr               Glu
```

aufweist.

3. Verfahren zur Herstellung eines cyclischen Polypeptids gemäß Anspruch 1, dadurch gekennzeichnet, daß man den das Polypeptid erzeugenden Streptomyces aureofaciens DSM 2790 oder einen seiner Varianten oder Mutanten in einem Fermentationsmedium im Submersverfahren kultiviert, das Polypeptid aus dem Mycel oder Kulturfiltrat in an sich bekannter Weise isoliert und reinigt.

4. Pharmazeutisches Präparat gekennzeichnet durch einen Gehalt an einem Polypeptid gemäß Anspruch 1.

5. Pharmazeutisches Präparat, dadurch gekennzeichnet, daß es das Polypeptid gemäß Anspruch 3 enthält.

6. Verwendung von Polypeptiden gemäß Anspruch 1 zur Hemmung der α-Amylase in vitro.

7. Verwendung von Polypeptiden gemäß Anspruch 1 zur Herstellung eines Arzneimittels zur Behandlung von Diabetes, Prädiabetes und Adipositas.

8. Verwendung von Polypeptiden gemäß Anspruch 1 zur Herstellung eines Diagnostikums, Reagenz oder Prophylaktikums gegen Karies.

9. Streptomyces aureofaciens DSM 2790 oder seine Varianten und Mutanten, welche Polypeptide gemäß Anspruch 1 produzieren.

**Patentansprüche für den Vertragsstaat: AT**

1. Verfahren zur Herstellung eines cyclischen Polypeptids, das aus 17 bis 200 Aminosäuren besteht und folgende Formel I aufweist

$$Z - S - Trp - Arg - Y \qquad\qquad I$$

in welcher bedeuten:

Z: Gln,

S: Ser,

Y: Tyr,

AS: eine Polypeptidkette aus 12 natürlichen Aminosäuren, die ihrerseits mit 2 Peptidketten substituiert ist,

wobei der α-Amylaseinaktivator Tendamistat (HOE 467) sowie der aus dem Kulturfiltrat von Streptomyces violaceoruber ATCC 31209 gewonnene α-Amylaseinhibitor ausgenommen sind, dadurch gekennzeichnet, daß man den das Polypeptid erzeugenden Streptomyces aureofaciens DSM 2790 in einem Fermentationsmedium im Submersverfahren kultiviert, das Polypeptid aus dem Mycel oder Kulturfiltrat in an sich bekannter Weise isoliert und reinigt.

2. Verfahren gemäß Anspruch 1, dadurch gekennzeichnet, daß man Streptomyces aureofaciens DSM 2790 kultiviert und aus dem Kulturfiltrat das Polypeptid AI—3688 der Formel Ia

```
Pro-Ala-Pro-Asp     Ser-Asp-Ala-Val-Thr-Val-Val
    |5              |            |            30              |
Ser                 Val-Cys-Cys-Gly-Asn-Arg                  Val
    |                   |10      25              |                |
Gly                 Glu                         Val            Gln         Ia
    |                   |                           |                |
                    Ser                         20Asp
Thr                 Phe    15                   Thr            35Tyr
    |                   |                           |                |
                    Gln— Ser— Trp— Arg—Tyr
Ala1                                                           Glu
```

isoliert und reinigt.

**Revendications pour les Etats contractants: BE CH DE FR GB IT LI LU NL SE**

1. Polypeptides cycliques, caractérisés en ce qu'ils sont constitués de 17 à 200 aminoacides et possèdent la formule I suivante

$$\overset{\displaystyle\frown\quad AS\quad\displaystyle\frown}{Z\ -\ S\ -\ Trp\ -\ Arg\ -\ Y}\qquad\qquad I$$

dans laquelle
Z: Gln,
S: Ser,
Y: Tyr,
AS: une chaîne polypeptidique de 12 aminoacides naturels, qui pour sa part est substituée par 2 chaînes peptidiques, à l'exclusion du désactivateur d'α-amylase Tendamistat (HOE 467) ainsi que de l'inhibiteur d'α-amylase obtenu à partir du filtrat de culture de *Streptomyces violaceoruber* ATCC 31209.

2. Polypeptide cyclique selon la revendication 1, caractérisé en ce qu'il possède la formule Ia

```
Pro-Ala-Pro-Asp     Ser-Asp-Ala-Val-Thr-Val-Val
    |5              |            |            30              |
Ser                 Val-Cys-Cys-Gly-Asn-Arg                  Val
    |                   |10      25              |                |
Gly                 Glu                         Val            Gln         Ia
    |                   |                           |                |
                    Ser                         20Asp
Thr                 Phe    15                   Thr            35Tyr
    |                   |                           |                |
                    Gln— Ser— Trp— Arg—Tyr
Ala1                                                           Glu
```

3. Procédé pour la production d'un polypeptide cyclique selon la revendication 1, caractérisé en ce que l'on cultive dans le procédé de culture submergée *Streptomyces aureofaciens* DSM 2790 ou l'un de ses variants ou mutants, producteur du polypeptide, dans un milieu de fermentation, et on isole et purifie d'une façon connue en soi le polypeptide à partir du mycélium ou du filtrat de culture.

4. Composition pharmaceutique caractérisée par une teneur en un polypeptide selon la revendication 1.

5. Composition pharmaceutique caractérisée en ce qu'elle contient le polypeptide selon la revendication 3.

6. Utilisation de polypeptides selon la revendication 1, pour l'inhibition de l'α-amylase in vitro.

7. Utilisation de polypeptides selon la revendication 1 pour la fabrication d'un médicament pour le traitement du diabète, de l'état prédiabétique et de l'adiposité.

8. Utilisation de polypeptides selon la revendication 1 pour la fabrication d'un agent de diagnostic, d'un réactif ou d'un prophylactique contre les caries.

9. *Streptomyces aureofaciens* DSM 2790 ou ses variants et mutants, qui produisent des polypeptides selon la revendication 1.

**Revendications pour l'Etat contractant: AT**

1. Procédé pour la préparation d'un polypeptide cyclique qui est constitué de 17 à 200 aminoacides et possède la formule I suivante

$$\overset{\displaystyle \frown \text{---AS---} \frown}{Z \ - \ S \ - \ Trp \ - \ Arg \ - \ Y} \qquad\qquad I$$

dans laquelle
Z: Gln,
S: Ser,
Y: Tyr,
AS: une chaîne polypeptidique de 12 aminoacides naturels, qui pour sa part est substituée par 2 chaînes peptidiques,
à l'exclusion du désactivateur d'α-amylase Tendamistat (HOE 467) ainsi que de l'inhibiteur d'α-amylase obtenu à partir du filtrat de culture de *Streptomyces violaceoruber* ATCC 31209, caractérisé en ce que l'on cultive dans le procédé de culture submergée *Streptomyces aureofaciens* DSM 2790 producteur du polypeptide, dans un milieu de fermentation, et on isole et purifie d'une façon connue en soi le polypeptide à partir du mycélium ou du filtrat de culture.

2. Procédé selon la revendication 1, caractérisé en ce que l'on cultive *Streptomyces aureofaciens* DSM 2790 et on isole et purifie le polypeptide AI—3688 de formule Ia

```
Pro-Ala-Pro-Asp     Ser-Asp-Ala-Val-Thr-Val-Val
 | 5            |         |              30       |
Ser       Val-Cys-Cys-Gly-Asn-Arg              Val
 |         |10        25      |                 |
Gly       Glu                Val               Gln       (Ia)
 |         |                  |                 |
 |         Ser              20Asp              |
Thr       Phe  15             Thr            35Tyr
 |         Gln— Ser—Trp —Arg—Tyr              |
Ala1                                          Glu
```

à partir du filtrat de culture.

**Claims for the Contracting States: BE CH DE FR GB IT LI LU NL SE**

1. A cyclic polypeptide which consists of 17 to 200 amino acids and has the following formula I

$$\overset{\displaystyle \frown \text{---AS---} \frown}{Z \ - \ S \ - \ Trp \ - \ Arg \ - \ Y} \qquad\qquad I$$

in which:
Z: denotes Gln,
S: denotes Ser,
Y: denotes Tyr and
AS: denotes a polypeptide chain of 12 naturally occurring amino acids, which is in turn substituted by 2 peptide chains,
excluding the α-amylase inactivator Tendamistat (HOE 467) and the α-amylase inhibitor isolated from the culture filtrate of Streptomyces violaceoruber ATCC 31209.

2. A cyclic polypeptide as claimed in claim 1, which has the formula Ia

```
Pro-Ala-Pro-Asp      Ser-Asp-Ala-Val-Thr-Val-Val
 |   5                |      /                 30          |
Ser            Val-Cys-Cys-Gly-Asn-Arg                 Val
 |              | 10      25              |              |
Gly            Glu                       Val           Gln       Ia
 |              |                         |             |
               Ser                      20Asp
 |              |                         |             |
Thr            Phe  15                   Thr          35Tyr
 |              |                         |             |
               Gln— Ser— Trp— Arg —Tyr
Ala1                                                   Glu
```

3. A process for the preparation of a cyclic polypeptide as claimed in claim 1, which comprises culturing, in a fermentation medium by the submersion method, Streptomyces aureofaciens DSM 2790 or one of its variants or mutants which produces the polypeptide, isolating the polypeptide from the mycelium or the culture filtrate in a manner which is known per se, and purifying it.

4. A pharmaceutical preparation containing a polypeptide as claimed in claim 1.

5. A pharmaceutical preparation containing the polypeptide as claimed in claim 3.

6. The use of a polypeptide as claimed in claim 1 for inhibiting α-amylase in vitro.

7. The use of a polypeptide as claimed in claim 1 for the preparation of a medicament for the treatment of diabetes, prediabetes and adiposity.

8. The use of a polypeptide as claimed in claim 1 for the preparation of a diagnostic agent, reagent or propylactic agent for caries.

9. Streptomyces aureofacians DSM 2790 or its variants or mutants which produce polypeptides as claimed in claim 1.

**Claims the for the Contracting State: AT**

1. A process for the preparation of a cyclic polypeptide which consists of 17 to 200 amino acids and has the following formula I

```
           ————AS————
         /             \
    Z  -  S  -  Trp  -  Arg  -  Y                I
```

in which:

Z: denotes Gln,

S: denotes Ser,

Y: denotes Tyr and

AS: denotes a polypeptide chain of 12 naturally occurring amino acids, which is in turn substituted by peptide chains,

excluding the α-amylase inactivator Tendamistat (HOE 467) and the α-amylase inhibitor isolated from the culture filtrate of Streptomyces violaceoruber ATCC 31209, which comprises culturing, in a fermentation medium by the submersion method, Streptomyces aureofaciens DSM 2790 which produces the polypeptide, isolating the polypeptide from the mycelium or the culture filtrate in a manner which is known per se, and purifying it.

2. The process as claimed in claim 1, wherein Streptomyces aureofaciens DSM 2790 is cultured and the polypeptide AI-3688 of the formula IA

```
Pro-Ala-Pro-Asp       Ser-Asp-Ala-Val-Thr-Val-Val
    |                  |                     30      |
    | 5                |                             |
Ser         Val-Cys-Cys-Gly-Asn-Arg                Val
    |           | 10        25       |               |
Gly         Glu                     Val             Gln         Ia
    |           |                     |               |
    |         Ser                   20Asp             |
Thr         Phe   15                 Thr           35Tyr
    |           |                     |               |
    |         Gln— Ser— Trp— Arg —Tyr                 |
Ala1                                               Glu
```

is isolated from the culture filtrate and purified.